(19) Europäisches Patentamt / European Patent Office / Office européen des brevets

(11) **EP 4 003 298 B1**

(12) **EUROPEAN PATENT SPECIFICATION**

(45) Date of publication and mention
of the grant of the patent:
**14.06.2023 Bulletin 2023/24**

(21) Application number: **20771921.2**

(22) Date of filing: **31.08.2020**

(51) International Patent Classification (IPC):
*A61K 9/127* (2006.01)     *A61K 9/00* (2006.01)
*A61K 31/12* (2006.01)     *A61K 36/9066* (2006.01)
*A61K 47/22* (2006.01)     *A61K 31/355* (2006.01)
*A61P 27/02* (2006.01)

(52) Cooperative Patent Classification (CPC):
(C-Sets available)
**A61K 9/0048; A61K 9/127; A61K 31/12;
A61K 31/355; A61K 36/9066; A61K 47/22;
A61P 27/02**                              (Cont.)

(86) International application number:
**PCT/IB2020/058100**

(87) International publication number:
**WO 2021/044283 (11.03.2021 Gazette 2021/10)**

(54) **EYE DROPS BASED ON A STERILIZABLE LIPOSOMAL FORMULATION BASED ON CURCUMIN AND OPHTHALMIC USE THEREOF**

AUGENTROPFEN AUF BASIS EINER STERILISIERBAREN LIPOSOMALEN FORMULIERUNG AUF BASIS VON KURKUMIN UND OPHTHALMISCHE VERWENDUNG DAVON

COLLYRE BASÉ SUR UNE FORMULATION LIPOSOMALE STÉRILISABLE À BASE DE CURCUMINE ET SON UTILISATION OPHTALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2019 IT 201900015408**

(43) Date of publication of application:
**01.06.2022 Bulletin 2022/22**

(73) Proprietor: **Offhealth S.p.A.**
**50144 Firenze (FI) (IT)**

(72) Inventors:
• **ZANINI, Alessandro**
**56017 San Giuliano Terme (PI) (IT)**
• **VELLANTE, Marco**
**62029 Tolentino (MC) (IT)**
• **SODO, Eugenio**
**00125 Rome (RM) (IT)**
• **CAVALLO, Giovanni**
**00122 Roma Ostia (RM) (IT)**

• **FOSCHINI, Fulvio**
**00124 Rome (RM) (IT)**
• **GIUVA, Roberto**
**62012 Civitanova Marche (MC) (IT)**

(74) Representative: **Sarpi, Maurizio et al**
**Studio Ferrario S.r.l.**
**Via Collina, 36**
**00187 Roma (IT)**

(56) References cited:
**CN-A- 1 824 255        CN-B- 102 008 439
CN-B- 102 379 850**

• **PESCOSOLIDO NICOLA ET AL: "Curcumin: therapeutic potential in ophthalmology", PLANTA MEDICA, GEORG THIEME VERLAG, DE, vol. 80, no. 4, 1 March 2014 (2014-03-01), pages 249-254, XP002789167, ISSN: 1439-0221, DOI: 10.1055/S-0033-1351074**

**(Cont. next page)**

Remarks:
The file contains technical information submitted after
the application was filed and not included in this
specification

(52) Cooperative Patent Classification (CPC): (Cont.)

C-Sets
**A61K 31/12, A61K 2300/00;**
**A61K 31/355, A61K 2300/00**

Remarks:
The file contains technical information submitted after
the application was filed and not included in this
specification

**Description**

FIELD OF THE INVENTION

The present invention relates to eye drops based on a liposomal formulation with curcumin and the ophthalmic use thereof. More in particular, the object of the invention is a liposomal formulation with curcumin which is stable over time, even without preservatives, and which is sterilizable by filtration on 0.2 um filter, so as to be usable as eye drops. In detail, the amount of curcumin varies from 0.025% to 0.050% by weight. Furthermore, the liposomal formulation includes the use of vitamin E - D-$\alpha$-tocopherol polyethylene glycol (TPGS) with the dual purpose of promoting the formation and stability of the liposomes and simultaneously providing a strong antioxidant effect. According to a peculiar feature of the invention, Vit-E/TGPS is present in an amount between 0.025 and 0.5, even better between 0.021 and 0.1 by weight with respect to the weight of the final composition, values below the ocular cytotoxicity threshold recognized in literature on the basis of scientific evidence and which lead to a solubilizing effect of the Curcumin only if the ratio with the latter is at least 10/1. (vitamin E-TPGS/curcumin). Said eye drops for use in the topical treatment of dry eye syndrome is an object of the invention as well.

BACKGROUND OF THE INVENTION

Curcumin is a very precious active natural constituent which is extracted from Turmeric root *(Curcuma fanga L.)*. Used for some time now, Turmeric is currently a very popular spice on our tables, Turmeric root is the main ingredient of curry, is called the golden spice, not only for the characteristic intense yellow-orange color with which it tinges the dishes in which it is present, but also for the numerous health benefits which it is capable of bringing. Turmeric has an important antioxidant action: Curcumin, one of the most precious constituents thereof, is capable of reaching the brain, as it is able to cross the blood-brain barrier. It is now known from the literature that curcumin has a clear anti-inflammatory activity. In fact, the action thereof in inhibiting and down-regulating the expression of cyclo-oxygenase 2 (COX-2) is well known. Some recent publications demonstrate the ability thereof to interact with a particular class of pain receptors, nociceptors. On the basis of these data, the soothing potential thereof would therefore be envisaged also for acute pain, often present in patients with various kinds of chronic diseases. According to these data, curcumin would desensitize the TRPAI nociceptor, even capable of mediating the analgesic effect of paracetamol. Since the action thereof in inhibiting and down-regulating the expression of COX-2, the preferential target of the anti-inflammatory molecule nimesulide, is known, a comparative pilot study was carried out on the ability of curcumin to relieve acute pain compared to paracetamol and nimesulide.

The anti-inflammatory potential of curcumin has also been shown to be effective against arthritis, known to be an inflammatory disease. All current approved arthritis medications are anti-inflammatory. Anti-TNF (tumor necrosis factor) therapy has recently been approved for this disease. Curcumin has been shown to be able to both stop TNF production and block the action of TNF. Applied topically, Curcumin has been shown to have activity against arthritis.

PRIOR ART

The main challenge when Curcumin use is desired is improving the bioavailability thereof, i.e., the absorption and use by the body. In recent years, various methods and strategies have been developed to optimize it. Furthermore, the poor solubility of Curcumin in water makes the use thereof in injectable form, and in the specific form of an eye drop, particularly difficult due to the impossibility of sterilizing the product by filtration even at low concentration. The situation is further complicated by the fact that the product cannot be sterilized by means of free-flowing steam sterilization at 121°C for at least 15 minutes at a pressure of 1 atm, as prescribed by the Pharmacopoeia, because the high temperature degrades the molecule.

Several products with specific features are known in the prior art, all aimed at making curcumin soluble and easily assimilable. Currently, the main mode of curcumin administration, as it is in powder form, is orally, known to have no toxicity, even at high dosages (Lao CD et al., 2006). In detail, we would like to highlight:

-    UniCunTM: To improve absorption after oral administration, Curcumin was complexed with a phospholipid system which increases the bioavailability thereof in the body 29 times, on average, compared to the uncomplexed dry extract (Cuomo J et al, 2011). This complex, present in UniCurTM, is called Curcumin Fitosoma®, which is an international patent. The solid form is the product Meriva®. In a study conducted by Di Pierro F et al., (Comparative evaluation of the pain-relieving properties of a lecithinized formulation of curcumin (Meriva®), nimesulide, and acetaminophen. Journal of Pain Research 2013:6201-205), it was possible to obtain a high oral-bioavailability curcumin for use in the treatment of pain and inflammation. The product showed a clear analgesic activity, comparable to that of paracetamol, but slightly lower than that of a therapeutic dose of nimesulide. The analgesic activity of Meriva® at lower doses (1.5 g) was less satisfactory. The onset time of the analgesic effect of Meriva® was later and the product took longer than nimesulide to reduce pain at both doses (1.5 and 2.0 g). As for the side effects, on the contrary, the gastric tolerability of Meriva® is significantly better than that of nimesulide and comparable to that of paracetamol. Taken together, these results demonstrate that the hypothesized analgesic properties of curcumin (in phytosomal form), have found further confirmation and clinical relevance in this study, at least as regards the 2 g dose. If on the one hand this dose is higher than that used in chronic inflammatory conditions (1.2 g/day), on the other hand the analgesic activity thereof could benefit from the reduction of the inflammatory response induced by curcumin, especially considering that the analgesia mechanisms mediated by the pain receptors are increased by the attenuation of inflammatory phenomena. In patients being treated for chronic inflammatory diseases, this translates into better acute pain control and provides a rationale capable of explaining the analgesic effects demonstrated by the phytosome curcumin.

- CURCUMINE REDOX: uses a unique and exclusive technology (NovaSOL® is a brand of AQUANOVA AG) due to which curcumin is more easily assimilable by the body, as it mimics the natural digestive processes. The matrix in which Curcumin is found is stable to gastric acidity and the Curcumin is released directly to the intestinal wall for maximum absorption. It is sufficient to use a single bead per day. The Novasol technique consists in solubilization by adding surfactants such as polysorbate 80 and polysorbate 20 at a high concentration (10/15%). The solubilization of the curcumin thus obtained makes it possible to administer curcumin orally.

However, the presence of surfactants gives the formulation an inherent toxicity which makes it unusable at the level of the ocular membranes.

It should further be considered that recent studies have led to the identification of negative effects due to the oral administration of curcumin, because the high absorption causes the precipitation of curcumin in the choledochus with consequent possible hepatitis. Therefore, in the hypothesis that the oral administration of curcumin can be useful for the well-being of the eye, in particular in diseases with an inflammatory component, these studies reinforce the idea that a topical administration of curcumin at the ocular level is the optimal solution.

Some studies have recently been presented (Bianco et al., 2013; Davis et al., 2017) in which nanocarriers were used to create a micellar nano-formulation to deliver curcumin to ocular tissues. In these studies, inorganic compounds such as Calixarene and Pluronic F-127 were used to obtain micelles capable of delivering curcumin on ocular tissues, in the form of eye drops and/or gel. Both of these studies use inorganic surfactants and although a micellar nano-formulation was obtained, there is no empirical evidence and/or indication that the resulting solution is sterilizable. Curcumin is known to be thermolabile, and therefore it is not possible to sterilize any curcumin-based formulation by free-flowing steam sterilization. Furthermore, the use of surfactants, cited in the studies reported above, mostly produces micellar solutions in the gel phase, which are difficult to sterilize by microfiltration.

PROBLEM TO SOLVE

As can be seen from the prior art, one of the limiting factors in the use of curcumin is the poor bioavailability thereof, particularly systemically, and solubility. Although there have been several attempts at local topical administration, one study has shown that only 3% of curcumin reaches the area to be treated. The low water solubility of curcumin makes the use thereof difficult. In the prior art and for those skilled in the art, various methods for the solubilization thereof are known, such as solubilization in alkaline buffer, in solvents such as DMSO (Dimethyl sulfoxide), but these are often toxic to cellular tissues and not applicable to eye tissues. The incorporation in nanoparticles for conveyance is known, but the use of surfactants for the stabilization of these particles often does not allow the use thereof in the ophthalmic field and does not allow a sterilization thereof by filtration, the only alternative to free-flowing steam sterilization at 121°C for 15 minutes at a pressure of 1 atm, which would degrade the curcumin.

To date, the need remains for a formulation which is easily sterilizable and which allows curcumin to be effectively conveyed to eye tissues, even for topical use.

In the Chinese patent document CN102008439, a curcumin-coated liposomal preparation belonging to the technical field of medical applications is described. In particular, a liposomal formulation is described comprising 10 mg of curcumin, 400 mg of lecithin and 10 mg of Vitamin E TPGS in 20 ml of phosphate buffer, capable of facilitating the oral absorption of poorly soluble drugs. Here, the ratio of vitamin E TPGS and Curcumin is 1/1, a ratio which does not give any solubilization of the Curcumin, but improves oral absorption due to Vitamin ETPGS.

Patent document CN102379850 describes a liposome aimed at the delivery of drugs, including curcumin, which crosses the human mucus barrier, particularly suitable for lungs and eyes. In particular, a liposomal formulation is described which comprises 0.1% by weight of curcumin, 1% by weight of Vitamin E TPGS and which is filtered through 0.22 micron filter membranes. In this case, the need to have a Vitamin E TPGS and Curcumin ratio of 10/1 is highlighted in order to have the curcumin solubilized, but this concentration of Vitamin E TPGS is high and cytotoxic to the eye.

Patent document CN 1824255 A discloses a zedoary turmeric oil solid lipid nanometer grain and its preparation method. Said solid lipid nanometer grain is a novel colloidal drug delivery system that can substitute emulsion, liposome and polymer nanometer grain and contains very medicinal phospholipid, emulsifier, solid lipid material antioxidant, etc. However no teaching is provided in relation to its possible ophthalmic use since in its examples the presence of Vit E TPGS is never less than 1%wt, that is high and cytotoxic for the eyes as in previous case.

As part of the research carried out by the Applicant on the development of a liposomal formulation of ophthalmic compositions based on curcumin for the topical treatment of inflammatory states of the ocular tissues, it has been found that numerous scientific studies show that high concentrations of vitamin E TGPS have a significant solubilizing effect on water-insoluble products, including Curcumin. Furthermore, it appears that the high concentration of Vitamin ETPGS paradoxically minimizes the carrier effect of liposomes formed by phospholipids and that when the concentration of Vitamin ETPGS (surfactant HLB = 13) increases, these can be destructured.

It should be added to what has just been said that with a concentration greater than 0.5% by weight, vitamin E TGPS has a cytotoxic (irritating) effect on the eye: this fact limits the use thereof at the ophthalmic level, being a substance with surfactant effect.

A formulation has therefore been experimentally defined where, only with a defined range of concentrations, vitamin E TPGS is compatible with the ocular use thereof, leaving the liposomes the primary role of carrier to convey the curcumin, and enhancing the oxidizing effect thereof: specifically, the best ratio of Vitamin E TPGS and Curcumin is 1/1 by weight and the ratio of Vitamin E TPGS and phospholipids is 1/20 by weight (0.050% Vitamin E TPGS / 1% Phospholipids)

The present invention therefore suggests a curcumin-based liposomal formulation according to the claims which is easily sterilizable by 0.2 micron filtration, where the presence of vitamin TPGS, between 0.05% and 0.5% by weight, even better between 0.05 and 0.100% by weight, allows the use thereof as eye drops, resulting below the cytotoxicity threshold mentioned above, while maintaining the carrier ability of the liposomes intact, the ratio between vitamin E TPGS and phospholipids being 1/20 or 1/10 respectively.

Advantageously, also due to the presence of Vitamin E - TPGS and the powerful antioxidant activity thereof, the object of the present invention is capable of reducing inflammatory phenomena.

OBJECT OF THE INVENTION

The first object of the present invention is the liposomal formulation of ophthalmic compositions based on curcumin for the topical treatment of inflammatory states of the ocular tissues, which is easily sterilizable by filtration.

It is another object of the present invention to provide a curcumin liposome ophthalmic composition, easily sterilizable by 0.2 micron filtration, which further comprises Vitamin E - TPGS, it is another object of the present invention to provide an easily sterilizable curcumin liposome ophthalmic composition, which further comprises Vitamin E-TPGS in which the weight ratio of curcumin and vitamin E TPGS is 1:1.

It is another object of the present invention to provide an easily sterilizable curcumin liposome ophthalmic composition which further comprises Vitamin E-TPGS, as a constituent of the liposomes themselves, in which the weight ratio of curcumin and vitamin E TPGS is 1:1, for use in the topical treatment of dry eye syndrome.

It is another object of the present invention to provide a curcumin liposome ophthalmic composition, which further comprises Vitamin E-TPGS, as a constituent of the liposomes themselves, in which the weight ratio of curcumin and vitamin E TPGS is 1:1, and where the liposomes consist of non-hydrogenated phospholipids, and have an average diameter less than 200 nm.

It is another object of the present invention to provide a curcumin liposome ophthalmic composition, which further comprises Vitamin E-TPGS, as a constituent of the liposomes themselves, in which the weight ratio of curcumin and vitamin E TPGS is 1:1, and where the liposomes consist of hydrogenated phospholipids, and have an average diameter less than 200 nm.

BRIEF DESCRIPTION OF THE DRAWINGS

Figure 1 shows the Gaussian distribution of the dimensions of the liposomes of the formulation object of the present invention, obtained using *light scattering* in intensity mode, after extrusion, before 0.2 um filtration;
Figure 2 shows the Gaussian distribution of the dimensions of the liposomes of the formulation object of the present invention, after 0.2 um filtration.

The two graphs demonstrate how the preparation and extrusion procedure used leads to a particle size clearly less than 200 nm, allowing the filterability of the product, while maintaining the initial curcumin titer.

DETAILED DESCRIPTION OF THE INVENTION

The fundamental component of the liposomal formulation according to the present invention is curcumin, extracted from Turmeric root and known for the antioxidant and anti-inflammatory properties thereof. Another component present in the formulation object of the present invention is vitamin E-TPGS, which improves the liposome filterability and, having itself an antioxidant power, acts by further enhancing the antioxidant ability of curcumin itself, moreover, stabilizing the formulation itself.

Experimental filtration tests were carried out on various possible formulations in which the concentration of curcumin varied from 0.1% w/w to 0.025% w/w and it was found that the formulations filterable at 0.2 microns were those in which this concentration ranged from 0.025% w/w to 0.050% by weight on the total weight.

Furthermore, the concentration of Vitamin E-TGPS ranged from 0.025% w/w to 0.5% w/w.

According to the present invention, in a preferred ophthalmic liposomal formulation the weight ratio of Vit E TPGS and curcumin is 1:1.

Preferably in this liposomal formulation the phospholipids are hydrogenated phospholipids with a phosphatidylcholine concentration between 80 and 90%.

A fundamental feature of the formulation thus obtained is that the liposomal population obtained has an average diameter less than 200 nm.

Furthermore, the possible formation of micro- and/or macro aggregates with dimensions greater than 200 nm must be prevented. In addition, excipients, buffering systems, and other agents commonly known to those skilled in the art and used in ophthalmic eye drops may be added to the liposomal formulation object of the present invention.

Furthermore, the formulation must be within the limits of the osmolarity (275 - 310 mOsmol/kg) and pH (7 - 7.4) parameters required for use as eye drops and known to those skilled in the art.

PHARMACEUTICAL FORMS AND DOSES OF THE OBJECT COMPOSITION

PHARMACEUTICAL COMPOSITIONS

In one aspect of the present invention, pharmaceutical liposomal compositions are provided comprising curcumin as a therapeutic agent, which may comprise Vitamin E-TGPS. Some embodiments may comprise one or more additional therapeutic agents, carriers and/or pharmacologically acceptable excipients known to those skilled in the art, which always allow the filterability of the eye drops.

Before reporting the examples of the prototypes which led to the development of a precise sterile liposomal eye drop by 0.2 micron filtration, we indicate the general procedure we used for the preparation of the liposomes containing curcumin below:

i) The non-hydrogenated phospholipids with curcumin are solubilized in organic solvent, such as ethanol, to allow the specific inclusion of the active ingredient in the phospholipids. The organic mixture consisting of the phospholipids and curcumin is vacuum dried until complete elimination of the solvents.

ii) The dry phospholipid paste containing curcumin is hydrated in water containing the buffer and other excipients, for a suitable hydration time, then it is homogenized with a shear homogenizer for a suitable period of time to obtain a mixture of very large phospholipid structures, with a size between 10-20 microns, which we will call Pre-liposomes in the following.

iii) The Pre-liposome solution is extruded at high pressure $9.119*10^7$ Pa to $1.013*10^8$ Pa (900-1000 atm) for at least 5 times to obtain the desired size smaller than 200 nm.

iv) The just extruded liposome solution is filtered in-line at 0.2 microns.

v) The liposome solution sterilized by 0.2 micron filtration is collected in a sterile container.

The procedure indicated above was carried out for all the formulations indicated below.

Some formulation examples are now reported for purely illustrative, non-limiting purposes of the invention, on which experimental tests have been carried out to verify the possibility of sterilization by means of 0.2 micron filtration and not by means of treatment at 121°C.

Other aspects, advantages or modifications within the scope of the invention will be apparent to those skilled in the art pertaining to the invention.

1) **Formulation 1:** Curcumin liposomes 0.1% Eye drops (not according to the claims)

| Raw materials | % w/w | CAS number |
|---|---|---|
| **Curcumin** | 0.100 | 458-37-7 |
| **Phospholipids S80*** | 1.000 | 93685-90-60 |
| Dibasic sodium phosphate dihydrate | 0.685 | 10028-24-7 |
| Monobasic sodium phosphate dihydrate | 0.225 | 13472-35-0 |
| Sodium chloride | 0.506 | 7647-14-5 |
| Distilled water as needed | 100 | - |
| **Sterile by filtration at 0.2 microns** | **no** | - |
| **Sterile by free-flowing steam at 121"C for 15 minutes at a pressure of 1 atm** | **no** | Product degradation - |
| **Osmolality mOsm/kg** | **275-310** | **-** |
| **PH** | **7-7.5** | **-** |

As seen in table 1, Formulation number 1 in curcumin liposomes at 0.1%, while containing non-hydrogenated phospholipids (specifically Phospholipon S80), i.e., represented by 80% phosphatidylcholine, with a phase transition between -5°C and 30°C, therefore particularly flexible, fluid, is not filterable at 0.2 microns: the curcumin concentration of 0.1% is the limiting factor.

To solve the problem of sterilization, which is the condition required for the development of eye drops, the possibility of steam sterilization at 121°C for 15 minutes at a pressure of 1 atm as prescribed by the pharmacopoeia has been hypothesized.

But the steam sterilization of the preparation led to the degradation of curcumin, which from an original yellow color became a dark green, which is the unequivocal index of a degradation effect caused by sterilization at 121°C for 20 minutes.

The browning of the solution was accompanied by a precipitation of the curcumin crystals over time. This indicated the destruction, at least partial, of the liposomes composed of non-hydrogenated phospholipids.

**2)Formulation 2:** Curcumin liposomes 0.05% eye drops (not according to the claims)

| Raw materials | % w/w | CAS number |
|---|---|---|
| **Curcumin** | 0.050 | 458-37-7 |
| **Phospholipids S80** | 1.000 | 93685-90-60 |
| Dibasic sodium phosphate dihydrate | 0.685 | 10028-24-7 |
| Monobasic sodium phosphate dihydrate | 0.225 | 13472-35-0 |
| Sodium chloride | 0.526 | 7647-14-5 |
| Distilled water as needed to | 100 | - |
| **Sterile by filtration at 0.2 microns** | **VERY DIFFICULT** | - |

Formulation number 2 in curcumin liposomes at 0.05% has a pH of 7.16 and an osmolarity (mOsmol/kg) of 287. Both values are within the acceptance limits (pH: 7-7.74; mOsmol/kg: 275-310). The formulation is only partially filterable at 0.2 microns, after a short period of time the filter is completely occluded, the product is therefore not sterilizable and therefore the use thereof as eye drops is precluded. It is apparent from this example that even with a decrease in the concentration of curcumin from the initial 0.100% to the concentration of 0.50%, the filterability of the product is very poor, especially if the industrial preparation of 100-200 liters of eye drops is considered. Like Formulation number 1, the attempt to steam sterilize the preparation led to the degradation of the curcumin and consequent breakdown of the liposomes with curcumin crystal deposits.

3) **Formulation 3:** Curcumin Liposomes 0.025% (not according to the claims)

| Raw materials | % w/w | CAS number |
|---|---|---|
| **Curcumin** | 0.025 | 458-37-7 |
| **Phospholipids S80** | 1.000 | - |
| Dibasic sodium phosphate dihydrate | 0.685 | 10028-24-7 |
| Monobasic sodium phosphate dihydrate | 0.225 | 13472-35-0 |
| Sodium chloride | 0.580 | 7647-14-5 |
| Distilled water as needed to | 100 | - |
| **Sterile by filtration at 0.2 microns** | **Possible but still VERY difficult** | - |

Formulation number 3 in curcumin liposomes at 0.025% has a pH of 7.2 and an osmolality (mOsmol/kg) of 295. Both values fall within the acceptance limits known in the state of the art.

Despite having decreased the curcumin concentration from the initial 0.05% to 0.025%, formulation number 3 was found to be filterable but still with great difficulty. Furthermore, the decrease in the curcumin concentration is additionally at the expense of the possible anti-inflammatory effect required.

In order to effectively solve the problem of 0.2 micron filtration, as the only sterilization method, the following formula was developed:

4) **Formulation 4:** Curcumin Liposomes 0.025% (not according to the claims)

| Raw materials | % w/w | CAS number |
|---|---|---|
| **Curcumin** | 0.025 | 458-37-7 |
| **Phospholipids S80** | 1.000 | - |
| **VITAMIN E-TPGS** | 0.500 | 9002-96-4 |
| Dibasic sodium phosphate dihydrate | 0.685 | 10028-24-7 |
| Monobasic sodium phosphate dihydrate | 0.225 | 13472-35-0 |
| Sodium chloride | 0.585 | 7647-14-5 |
| Distilled water as needed to | 100 | - |
| **Sterile by filtration at 0.2 microns** | **Filterable** | - |

Formulation number 4 in curcumin liposomes at 0.025% has a pH of 7.3 and an osmolality (mOsmol/kg) of 292. Both values are within the acceptance limits (pH: 7-7.4; mOsmol/kg: 275-310).

Formulation number 4 was found to be filterable, having decreased the concentration to 0.025% from the initial 0.05%, as in formulation number 3, but with the addition of Vitamin E-TPGS in the formulation, outside the liposomes.

Vitamin E-TPGS at a concentration of 0.5% is added in step ii) of the preparation process of the formulation as reported above, during the hydration of the phospholipid paste containing curcumin in water, and before the homogenization thereof. Thereby, the presence of Vitamin E-TGPS is ensured especially on the outside of the liposomes, improving the filterability of the curcumin liposomes.

Although Vitamin E-TGPS has a significant effect on the liposome filterability and ensures a synergistic antioxidant effect of the curcumin itself, it could have, at a concentration of 0.5%, a possible toxicity at the ocular surface, as in addition to being a vitamin it is also a surfactant. To this end, according to the invention, a curcumin formulation at 0.025% with Vitamin E-TPGS at a concentration lower than 0.1% was developed to limit the possible undesirable effects. However, to ensure the fluidifying activity thereof, vitamin E-TGPS (with a concentration of 0.1%) is inserted inside the liposomal structure, advantageously adding it in the solubilization phase of the non-hydrogenated phospholipids and curcumin, i.e., step i) of the preparation method of the previously described formulation. The formulation thus obtained is as follows:

**Formulation 5.** Curcumin liposomes 0.025%/Vitamin E - TPGS 0.100% (inside the liposomal structure)

| Raw materials | % w/w | CAS number |
|---|---|---|
| **Curcumin** | 0.025 | 458-37-7 |
| **Phospholipids S80** | 1.000 | - |
| **Vitamin E TPGS** | 0.100 | 9002-96-4 |
| Dibasic sodium phosphate dihydrate | 0.685 | 10028-24-7 |
| Monobasic sodium phosphate dihydrate | 0.225 | 13472-35-0 |
| Sodium chloride | 0.570 | 7647-14-5 |
| Distilled water as needed to | 100 | - |
| **0.2 micron sterile filtration** | **Very good** | - |

Formulation number 5 in curcumin liposomes at 0.025% has a pH of 7.22 and an osmolality (mOsmol/kg) of 280. Both values are within the acceptance limits (pH: 7-7.4; mOsmol/kg: 275-310). Furthermore, the average diameter of the liposomal particles is less than 200 nm. The liposome formulation of curcumin at 0.025% and Vitamin E-TPGS at 0.100% (as a constituent of the liposomes themselves) is filterable at 0.2 microns; the product is therefore sterilizable and thus allowing the use thereof as eye drops without preservatives, thereby making the industrial development thereof viable.

Following the idea of including Vitamin E-TGPS inside the liposomes, an attempt was made to reduce the concentration thereof from 0.1% to 0.050%, and simultaneously increase the concentration of curcumin from 0.025% to 0.050%, in order to ensure greater anti-inflammatory efficacy at the ocular level.

The following formulation is obtained:

**Formulation** 6: Curcumin liposomes 0.05%/Vitamin E - TPGS 0.05% (inside the liposomal structure)

| Raw materials | % w/w | CAS number |
|---|---|---|
| **Curcumin** | 0.050 | 458-37-7 |
| **Phospholipids S80** | 1.000 | 803-76-0 |
| **Vitamin E TPGS** | 0.050 | 9002-96-4 |
| Dibasic sodium phosphate dihydrate | 0.685 | 10028-24-7 |
| Monobasic sodium phosphate dihydrate | 0.225 | 13472-35-0 |
| Sodium chloride | 0.570 | 7647-14-5 |
| Distilled water as needed to | | - |
| **0.2 micron sterile filtration** | **yes** | - |

Formulation number 6 in curcumin liposomes at 0.050% has a pH of 7.17 and an osmolality (mOsmol/kg) of 285. Both values are within the acceptance limits (pH: 7-7.4; mOsmol/kg: 275-310). Furthermore, the average diameter of the liposomal particles is less than 200 nm. The liposome formulation of curcumin at 0.050% and Vitamin E-TPGS at 0.05% (as a constituent of the liposomes themselves) is well filterable at 0.2 microns, allowing the use thereof as eye drops, without adding preservatives.

Having obtained an excellent result, the previous formulation was further improved, replacing the non-hydrogenated phospholipids (S80), having a phase transition at low temperature (-5 and 30°C) which is very flexible and therefore easily filterable, with hydrogenated phospholipids (specifically PHOSPOLIPON 90H, i.e., with a concentration of phosphatidylcholine between 80-90%), much more rigid having a phase transition (45-55°C) and, therefore, less flexible and fluid and thus difficultly filterable at 0.2 microns.

This choice was made to improve the overall stability of the formulation, as although non-hydrogenated phospholipids are more easily filterable, they are less stable than hydrogenated phospholipids: in fact, the latter can degrade over time due to oxygen and even temperature with possible color changes and possible presence of peroxides. Furthermore, due to the intrinsic rigidity thereof, they are more resistant carriers preventing the release of the active ingredients over time.

In consideration of the above, we report the following formulation number 7 here, which has been studied from a pharmacological point of view.

**Formulation Number 7:** Curcumin liposomes 0.05%/Vitamin E - TPGS 0.05% with hydrogenated phospholipids (inside the liposomes)

| Raw materials | % w/w | CAS number |
| --- | --- | --- |
| **Curcumin** | 0.050 | 458-37-7 |
| **Phospholipids 90H** | 1.000 | 97281-48-6 |
| **VITAMIN E-TPGS** | 0.050 | 9002-96-4 |
| Dibasic sodium phosphate dihydrate | 0.695 | 10028-24-7 |
| Monobasic sodium phosphate dihydrate | 0.225 | 13472-35-0 |
| Sodium chloride | 0.570 | 7647-14-5 |
| Distilled water as needed to | | - |
| **0.2 micron sterile filtration** | **yes** | - |

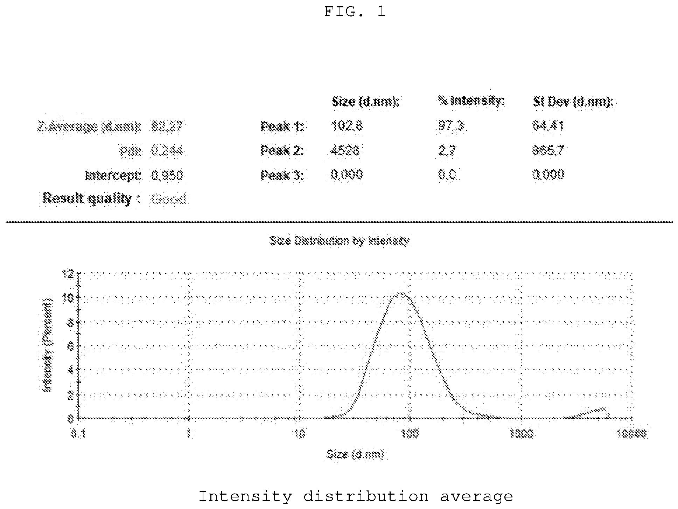

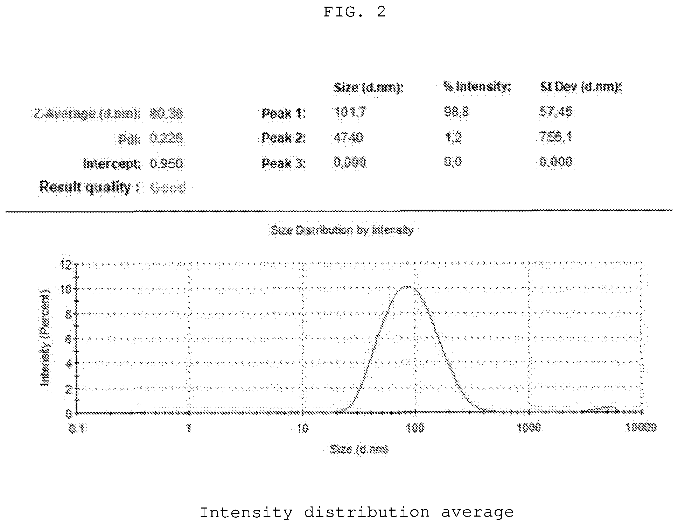

Formulation number 7 in curcumin liposomes at 0.050% has a pH of 7.10 and an osmolality (mOsmol/kg) of 285. Both values are within the acceptance limits (pH: 7-7.4; mOsmol/kg: 275-310). Furthermore, the average diameter of the liposomal particles is less than 200 nm, as shown in Figure 1. Despite the replacement of the non-hydrogenated phospholipids (more flexible and filterable) with hydrogenated ones (more rigid and less filterable), the liposome formulation of curcumin at 0.050% and Vitamin E-TPGS at 0.05% (as a constituent of the liposomes themselves) is still well filterable at 0.2 microns, as seen in Figure 2, therefore sterilizable and usable for use as eye drops.

Based on the above, formulation number 7 was therefore assayed in the experimental tests described below, aimed at evaluating the anti-inflammatory efficacy thereof.

It is important to note that in the pharmacological experiment carried out illustrated below, a cell viability assay is reported with excellent results demonstrating that the strategy of containing the concentration of the surfactant VITAMIN E TPGS as much as possible was appropriate and correct (see the following table 2) which is accompanied by an excellent anti-inflammatory ability (see following table 3) (inhibition of cytokines).

In addition, formulation number 7 was further improved by replacing the phosphate buffer, used in all the previous formulations 1 to 7, with the borate buffer, which is known to be much more accepted at the ocular level.

The formulation developed is the following:

**Formulation 8:** Curcumin liposomes 0.05%/Vitamin E - TPGS 0.05% with hydrogenated phospholipids (inside the liposomes)

| Raw materials | % w/w | CAS number |
| --- | --- | --- |
| Curcumin | 0.050 | 458-37-7 |
| Phospholipids 90H | 1.000 | 97281-48-6 |

(continued)

| Raw materials | % w/w | CAS number |
|---|---|---|
| VITAMIN E-TPGS | 0.050 | 9002-96-4 |
| Boric acid | 0.775 | 10043-35-3 |
| SODIUM TETRABORATE DECAHYDRATE | 0.125 | 1303-96-4 |
| Sodium chloride | 0.450 | 7647-14-5 |
| Distilled water as needed to | | - |
| **0.2 micron sterile filtration** | **yes** | - |

Formulation number 8 in curcumin liposomes at 0.050% has a pH of 7.24 and an osmolality (mOsmol/kg) of 286. Both values are within the acceptance limits (pH: 7-7.4; mOsmol/kg: 275-310). Furthermore, the average diameter of the liposomal particles is less than 200 nm, making it filterable at 0.2 microns, therefore sterilizable and usable for use as eye drops.

EXPERIMENTAL PART

IN VITRO ASSESSMENT OF THE ANTI-INFLAMMATORY POTENTIAL OF THE LIPOSOMAL OPHTHALMIC FORMULATION

**FORMULATION NUMBER 7**

To verify the anti-inflammatory effect of the curcumin-based liposomal ophthalmic formulation object of the present invention, an experimental test was carried out, evaluating in vitro, in detail, the anti-inflammatory potential on reconstructed human epidermis.

The aim of the test was to quantitatively ascertain the effects of the formulation in question in the protection of epithelial cells and in the inhibition of the inflammatory reaction caused by a moderate irritant such as sodium lauryl sulfate (SLS) by means of an in vitro model of multilayer epithelial cells. The inhibition of SLS mediated release of the cytokine IL-1$\alpha$ following the exposure of the formulation in question and by comparison with untreated epidermis was evaluated by means of an ELISA assay. The assay included a prime MTT assay at the end of the incubation: cell survival assay using the reconstructed in vitro 3D artificial human epidermis to assess the cytotoxic potential of the sample. Furthermore, there will be inhibition of SLS mediated release of inflammatory mediators (IL-1$\alpha$).

Test Procedures

I. Cell model

The in vitro test systems used consist of an artificial three-dimensional system of the human epidermis. A reconstructed model of artificial human skin was prepared comprising normal human epidermal keratinocytes, which was grown as an integrated three-dimensional cell culture model, perfectly mimicking human skin *in vitro.* The model exhibits normal barrier functions (presence of a well-differentiated stratum corneum). It was provided by CELLSYSTEMS (Troisdorf, lot 100-AH1244-1).

II. Treatment and exposure

The epidermis was pre-treated for 60 minutes with 0.25% SLS and was obtained in order to induce the synthesis and release of IL-1$\alpha$. Later 30 $\mu$l of the formulation object of the invention was applied on two portions of the epidermis in two replicates, and the exposure was carried out for 24 hours at 37°C, 5% $CO_2$. As a negative control, two epidermal units were treated with phosphate buffer, while the epidermis treated for 60 minutes with 0.25% SLS was used as a positive control. The assay was performed in two replicates. At the end of the exposure period, the sample formulation and controls were removed by washing with phosphate buffer (PBS) and the MTT assay was performed to assess cell survival. For the IL 1$\alpha$ test, the culture medium was collected at 6 and 24 hours. Only the culture medium from the PBS-treated units and mediums from sample-treated, non-irritated epidermal units were used as negative controls; while the mediums of the 2 units treated with SLS 0.25% were used as positive controls.

III. MTT cell viability test

The epidermal units are treated with 1 mg of MD (3-4,5-dimethyl-thiazol-2-yl)-2,5-diphenyl-tetrazolium bromide) solution for 3 hours at 37°C. The solution is then removed and replaced with isopropanol, with a further 2 hours of incubation at room temperature; 2 aliquots of each sample are transferred to a 96-well plate for reading. The absorbance is read at the 570 nm wavelength with a colorimeter (Tecan Sunrise remote model) provided with a microplate reader.

IV. IL-1$\alpha$ assay

In this study IL-1$\alpha$ was determined in the culture medium of treated and untreated epidermis, using a direct ELIS assay (enzyme-linked immuno-absorbent assay). The colorimetric signal is directly proportional to the amount of cytokine in the culture medium. The samples are read at 450 nm. The detection limit is less than 10 pg/ml.

V. Expression of the results.

The results are expressed in terms of cell viability:

```
% cell viability = [OD(570 nm) test sample/OD (570
nm) CN] x 100
```

IL-1$\alpha$

Cytokine concentration is determined using a standard curve. The inhibition of IL-1$\alpha$ release was determined as: % of IL-1$\alpha$ inhibition:

```
100-(pg/ml    IL-1α    released    from    irritated
samples/pg/ml IL-1α positive control)*100
```

VI. Method acceptance criteria

MTT test/dose

for the negative control (CN): OD value between 1.0-3.0.
standard deviation must be ≤18%
for the positive control (CP): the average viability (expressed as % of the NC) must be <70%
standard deviation must be ≤ 18%
for the sample: the standard deviation must be <18%.

**Table 1.** Criteria for assay acceptance.

| MTI | | VALUE | LIMITS | RESULTS |
|---|---|---|---|---|
| Positive control | Average viability | 65.79 | <70% | Compliant |
| | Standard Dev. % | 0.45 | S18% | Compliant |
| Negative Control | Average viability | 1.54 | 1.0-3.0 | Compliant |
| | Standard Dev. % | 6.91 | ≤ 18% | Compliant |
| Sample: Standard Dev. % | | 9.16 | ≤ 18% | Compliant |

Table 2. Average cell viability values (%)

| MTT | % Average cell viability (ds) |
|---|---|
| Formulation no. 7<br>NLL-103 (Niolip S.r.l.) - Ophthalmic solution - Sample 18-3433<br>Lot/Batch: laboratory prepared on 02/01/2018 | 99.47 (9.16) |
| Formulation no. 7<br>NLL-1 03 (Niolip S.r.l.) - Ophthalmic solution - Sample 18-3433<br>Lot/Batch: laboratory prepared on 02/01/2018 + SLS 0.25% | 86.83 (3.05) |
| SLS 0.25% Positive Control (CP) | 65.79 (0.45) |
| Negative Control (PBS) | 100.00 (6.91) |

Table 3. Amount of IL-1$\alpha$ expressed in pg/ml

| | IL-1$\alpha$ pg/ml | | | |
|---|---|---|---|---|
| | 6h | | | 24h |
| | pg/ml average | % Inhibition | pg/ml averag e | % inhibition |
| Formulation no. 7<br>NLL-103 (Niolip S.r.l.) - Ophthalmic solution - Sample 18-3433<br>Lot/Batch: laboratory prepared on 02/01/2018 | 5.34 | | 14.00 | 46.60 |
| Formulation no. 7<br>NLL-103 (Niolip S.r.l.) - Ophthalmic solution - Sample 18-3433<br>Lot/Batch: laboratory | 39.13 | 50.57 | 124.88 | |
| prepared on 02/01/2018 + SLS 0.25% | | | | |
| SLS 0.25% | 79.17 | | 233.86 | |
| Negative control | 3.97 | | 10.74 | |

Results

On the basis of the results shown here, the sample of Formulation number 7 NLL-103 (Niolip S.r.l.) - ophthalmic solution shows anti-inflammatory activity *in vitro.* Already after 6 hours of incubation, a reduction in the release of IL-1$\alpha$ (50.57%) is highlighted.

CONCLUSIONS

The anti-inflammatory efficacy of the liposomal formulation according to the invention was determined by standard experimental tests conducted in cell cultures of reconstructed human epidermis. The data thus obtained were used to determine the above formulations as the optimal dose ranges for the use of the composition in humans.

**Claims**

1.  An ophtalmic liposomal formulation with curcumin and vitamin E-D-$\alpha$- polyethylene glycol (TPGS), **characterized in that** the amount of curcumin varies from 0.025% to 0.050% by weight, by total weight, so that this formulation is filterable and sterilizable at 0.2 microns, and the vitamin E-D-$\alpha$-polyethylene glycol (TPGS) is inserted inside the liposomal structure in an amount between 0.05 and 0.5% by weight, with the dual purpose of promoting the formation and filterability of the liposomes and simultaneously providing a general stability of the formulation and a strong antioxidant effect, said formulation being within the acceptance limits of the osmolality of 275-310 mOsM/kg and the pH 7-7.4.

2. The ophthalmic liposomal formulation according to claim 1, **characterized in that** the curcumin and vitamin E TPGS are both at a concentration of 0.050% by weight with respect to the total weight of the formulation.

3. The ophthalmic liposomal formulation according to claims 1 and 2, **characterized in that** it further comprises excipients, buffering systems, and other agents commonly known to those skilled in the art and used in ophthalmic eye drops to allow the specific inclusion of the active ingredient in phospholipids.

4. The ophthalmic liposomal formulation according to claims 2 and 3, having the following composition:

|  | % w/w | CAS Number: |
|---|---|---|
| **Curcumin** | 0.050 | 458-37-7 |
| **Phospholipids S80** | 1.000 | 8030-70-0 |
| **VITAMIN E-TPGS** | 0.050 | 9002-96-4 |
| Dibasic sodium phosphate dihydrate | 0.685 | 10028-24-7 |
| Monobasic sodium phosphate dihydrate | 0.225 | 13472-35-0 |
| Sodium chloride | 0.526 | 7647-14-5 |
| Distilled water as needed to | 100 | |

which is sterile by filtration at 0.2 microns, with an osmolality of 285 mOsm/kg and a pH of 7.17, where the phospholipids (S80) are not hydrogenated, and the dibasic sodium phosphate dihydrate is used as a buffering system.

5. The ophthalmic liposomal formulation according to claim 2 and 3 having the following composition:

|  | % w/w | CAS number |
|---|---|---|
| **Curcumin** | 0.050 | 458-37-7 |
| **Phospholipids 90H** | 1.000 | 97281-48-0 |
| **VITAMIN E-TPGS** | 0.050 | 9002-96-4 |
| Dibasic sodium phosphate dihydrate | 0.685 | 10028-24-7 |
| Monobasic sodium phosphate dihydrate | 0.225 | 13472-35-0 |
| Sodium chloride | 0.570 | 7647-14-5 |
| Distilled water as needed to | 100 | |
|  |  |  |

which is sterile by filtration, with an osmolality of 286 mOsM/kg and a pH of 7.24, where the phospholipids (90H) are hydrogenated, and the dibasic sodium phosphate dihydrate is used as a buffering system.

6. The ophthalmic liposomal formulation according to claims 2 and 3 having the following composition:

|  | % w/w | CAS Number: |
|---|---|---|
| **Curcumin** | 0.050 | 458-37-7 |
| **Phospholipids 90H** | 1.000 | 97281-48-0 |
| **VITAMIN E-TPGS** | 0.050 | 9002-96-4 |
| Boric acid | 0.775 | 10043-35-3 |
| SODIUM TETRABORATE DECAHYDRATE | 0.125 | 1303-96-4 |
| Sodium chloride | 0.450 | 7647-14-5 |
| Distilled water as needed to | 100 | |

which is sterile by filtration, with an osmolality of 286 mOsM/kg and a pH of 7.24, where the phospholipids (90H) are hydrogenated, and the boric acid is used as a buffering system.

7. The ophthalmic liposomal formulation according to any one of the preceding claims for use as eye drops, after sterilization by filtration.

8. The ophthalmic liposomal formulation according to any one of the preceding claims for use after sterilization by filtration, as eye drops for the treatment of dry eye syndrome.

9. A method for preparing the ophthalmic liposomal formulation with curcumin and Vit. E TPGS of any one of claims 1 to 6, **characterized in that** it comprises the following operating steps:

i) Solubilization of phospholipids with curcumin and Vit E TPGS in an organic solvent for the specific inclusion of the active ingredient and of Vit. E TPGS in the phospholipids, and vacuum drying of the organic mixture consisting of the phospholipids, curcumin and Vit.E TPGS up to complete elimination of the solvents.
ii) The dry phospholipid paste thus obtained is hydrated in water containing the buffer and other excipients for a suitable hydration time, with subsequent homogenization with a shear homogenizer for a suitable period of time in order to obtain a mixture of Pro-liposomes.
iii) Extrusion of the solution of Pro-liposomes at high pressure $9.119*10^7$ Pa to $1.013*10^8$ Pa ( 900-1000 atm ) for at least 5 times to obtain the desired size less than 200 nm.
iv) 0.2 micron in-line filtration of the just extruded liposome solution.
v) Collection of the liposome solution sterilized by 0.2 micron filtration in a sterile container.

10. The method for preparing the ophthalmic liposomal formulation according to claim 9, **characterized in that** in step i) hydrogenated phospholipids are used.

11. The method for preparing the ophthalmic liposomal formulation according to claim 9, **characterized in that** in step i) non-hydrogenated phospholipids are used.

12. An ophthalmic liposomal composition of curcumin with vitamin E-TPGS intercalated from the beginning in lipid film to obtain greater protection, obtained according to the method of claim 9, for use after 0.2 $\mu$m filtration, as eye drops for the topical treatment of dry eye syndrome.

**Patentansprüche**

1. Ophthalmische liposomale Formulierung mit Curcumin und Vitamin E-D-$\alpha$-Polyethylenglykol (TPGS), **dadurch gekennzeichnet, dass** die Menge an Curcumin von 0,025 bis 0,050 Gew.-%, bezogen auf das Gesamtgewicht, variiert, so dass diese Formulierung bei 0,2 Mikron filtrierbar und sterilisierbar ist, und das Vitamin E-D-$\alpha$-Polyethylenglykol (TPGS) in die liposomale Struktur in einer Menge zwischen 0,05 und 0,5 Gew.-% eingebracht wird, mit dem doppelten Zweck, die Bildung und Filtrierbarkeit der Liposomen zu fördern und gleichzeitig eine allgemeine Stabilität der Formulierung und eine starke antioxidative Wirkung bereitzustellen, wobei die Formulierung innerhalb der Akzeptanzgrenzen der Osmolalität von 275-310 mOsM/kg und des pH-Werts von 7-7,4 liegt.

2. Ophthalmische liposomale Formulierung gemäß Anspruch 1, **dadurch gekennzeichnet, dass** das Curcumin und das Vitamin E TPGS beide in einer Konzentration von 0,050 Gew.-%, bezogen auf das Gesamtgewicht der Formulierung, vorliegen.

3. Ophthalmische liposomale Formulierung gemäß den Ansprüchen 1 und 2, **dadurch gekennzeichnet, dass** sie ferner Hilfsstoffe, Puffersysteme und andere Mittel umfasst, die dem Fachmann allgemein bekannt sind und in ophthalmischen Augentropfen verwendet werden, um den spezifischen Einschluss des Wirkstoffs in Phospholipiden zu ermöglichen.

4. Ophthalmische liposomale Formulierung gemäß den Ansprüchen 2 und 3, die die folgende Zusammensetzung aufweist:

|  | Gew.-% | CAS-Nummer: |
|---|---|---|
| **Curcumin** | 0,050 | 458-37-7 |
| **Phospholipide S80** | 1,000 | 8030-70-0 |
| **VITAMIN E-TPGS** | 0,050 | 9002-96-4 |
| Zweibasisches Natriumphosphat-Dihydrat | 0,685 | 10028-24-7 |
| Monobasisches Natriumphosphat-Dihydrat | 0,225 | 13472-35-0 |
| Natriumchlorid | 0,526 | 7647-14-5 |
| Destilliertes Wasser nach Bedarf, für | 100 | |

die durch Filtration bei 0,2 Mikron steril ist, mit einer Osmolalität von 285 mOsm/kg und einem pH-Wert von 7,17, wobei die Phospholipide (S80) nicht hydriert sind, und das zweibasische Natriumphosphat-Dihydrat als Puffersystem verwendet wird.

5. Ophthalmische liposomale Formulierung gemäß den Ansprüchen 2 und 3, die die folgende Zusammensetzung aufweist:

|  | Gew.-% | CAS-Nummer: |
|---|---|---|
| **Curcumin** | 0,050 | 458-37-7 |
| **Phospholipide 90H** | 1,000 | 97281-48-0 |
| **VITAMIN E-TPGS** | 0,050 | 9002-96-4 |
| Zweibasisches Natriumphosphat-Dihydrat | 0,685 | 10028-24-7 |
| Monobasisches Natriumphosphat-Dihydrat | 0,225 | 13472-35-0 |
| Natriumchlorid | 0,570 | 7647-14-5 |
| Destilliertes Wasser nach Bedarf, für | 100 | |
|  |  |  |

die durch Filtration steril ist, mit einer Osmolalität von 286 mOsm/kg und einem pH-Wert von 7,24, wobei die Phospholipide (90H) hydriert sind, und das zweibasische Natriumphosphat-Dihydrat als Puffersystem verwendet wird.

6. Ophthalmische liposomale Formulierung gemäß den Ansprüchen 2 und 3, die die folgende Zusammensetzung aufweist:

|  | Gew.-% | CAS-Nummer: |
|---|---|---|
| **Curcumin** | 0,050 | 458-37-7 |
| **Phospholipide 90H** | 1,000 | 97281-48-0 |
| **VITAMIN E-TPGS** | 0,050 | 9002-96-4 |
| Borsäure | 0,775 | 10043-35-3 |
| NATRIUMTETRABORAT-DECAHYDRAT | 0,125 | 1303-96-4 |
| Natriumchlorid | 0,450 | 7647-14-5 |
| Destilliertes Wasser nach Bedarf, für | 100 | |

die durch Filtration steril ist, mit einer Osmolalität von 286 mOsm/kg und einem pH-Wert von 7,24, wobei die Phospholipide (90H) hydriert sind, und die Borsäure als Puffersystem verwendet wird.

**7.** Ophthalmische liposomale Formulierung gemäß einem der vorhergehenden Ansprüche zur Verwendung als Augentropfen, nach Sterilisation durch Filtration.

**8.** Ophthalmische liposomale Formulierung gemäß einem der vorhergehenden Ansprüche zur Verwendung nach Sterilisation durch Filtration als Augentropfen zur Behandlung des Syndroms des trockenen Auges.

**9.** Verfahren zum Herstellen der ophthalmischen liposomalen Formulierung mit Curcumin und Vit. E TPGS gemäß einem der Ansprüche 1 bis 6, **dadurch gekennzeichnet, dass** es die folgenden Arbeitsschritte umfasst:

i) Lösen von Phospholipiden mit Curcumin und Vit. E TPGS in einem organischen Lösungsmittel zum spezifischen Einschluss des Wirkstoffs und von Vit. E TPGS in die Phospholipide, und Vakuumtrocknen des organischen Gemisches, das aus den Phospholipiden, Curcumin und Vit. E TPGS besteht, bis zur vollständigen Entfernung der Lösungsmittel.
ii) Die so erhaltene trockene Phospholipidpaste wird in Wasser, das Puffer und andere Hilfsstoffe enthält, für eine geeignete Hydratationszeit hydratisiert, mit anschließender Homogenisierung mit einem Scher-Homogenisierer für eine geeignete Zeitspanne, um eine Mischung von Pro-Liposomen zu erhalten.
iii) Extrusion der Lösung von Pro-Liposomen bei hohem Druck $9,119*10^7$ Pa bis $1,013*10^8$ Pa (900-1000 atm) mindestens fünfmal, um die gewünschte Größe von weniger als 200 nm zu erhalten.
iv) Inline-Filtration der gerade extrudierten Liposomenlösung mit 0,2 Mikron.
v) Auffangen der durch 0,2-Mikron-Filtration sterilisierten Liposomenlösung in einem sterilen Behälter.

**10.** Verfahren zum Herstellen der ophthalmischen liposomalen Formulierung gemäß Anspruch 9, **dadurch gekennzeichnet, dass** in Schritt i) hydrierte Phospholipide verwendet werden.

**11.** Verfahren zum Herstellen der ophthalmischen liposomalen Formulierung gemäß Anspruch 9, **dadurch gekennzeichnet, dass** in Schritt i) nicht-hydrierte Phospholipide verwendet werden.

**12.** Ophthalmische liposomale Zusammensetzung von Curcumin mit Vitamin E-TPGS, das von Anfang an in den Lipidfilm interkaliert ist, um einen größeren Schutz zu erhalten, die gemäß dem Verfahren nach Anspruchs 9, zur Verwendung nach 0,2 um Filtration als Augentropfen für die topische Behandlung des Syndroms des trockenen Auges erhalten wird.

**Revendications**

**1.** Formulation liposomale ophtalmique contenant de la curcumine et de la vitamine E-D-$\alpha$-polyéthylène glycol (TPGS), **caractérisée en ce que** la quantité de curcumine varie de 0,025 % à 0,050 % en poids, en poids total, de sorte que cette formulation soit filtrable et stérilisable à 0,2 micron, et la vitamine E-D-$\alpha$-polyéthylène glycol (TPGS) est insérée dans la structure liposomale en une quantité entre 0,05 et 0,5 % en poids, dans le double but de favoriser la formation et la filtrabilité des liposomes et d'assurer simultanément une stabilité générale de la formulation et un fort effet antioxydant, ladite formulation se situant dans les limites d'acceptation de l'osmolalité de 275 à 310 mOsM/kg et du pH de 7 à 7,4.

**2.** Formulation liposomale ophtalmique selon la revendication 1, **caractérisée en ce que** la curcumine et la vitamine E TPGS sont toutes deux à une concentration de 0,050 % en poids par rapport au poids total de la formulation.

**3.** Formulation liposomale ophtalmique selon les revendications 1 et 2, **caractérisée en ce qu'**elle comprend en outre des excipients, des systèmes tampons et d'autres agents communément connus de l'homme de l'art et utilisés dans des gouttes ophtalmiques pour permettre l'inclusion spécifique du principe actif dans les phospholipides.

**4.** Formulation liposomale ophtalmique selon les revendications 2 et 3, ayant la composition suivante :

| | % p/p | Numéro CAS : |
|---|---|---|
| Curcumine | 0,050 | 458-37-7 |
| Phospholipides S80 | 1,000 | 8030-70-0 |
| VITAMINE E-TPGS | 0,050 | 9002-96-4 |

(suite)

|  | % p/p | Numéro CAS : |
|---|---|---|
| Orthophosphate disodique dihydraté | 0,685 | 10028-24-7 |
| Orthophosphate monosodique dihydraté | 0,225 | 13472-35-0 |
| Chlorure de sodium | 0,526 | 7647-14-5 |
| Eau distillée si nécessaire pour | 100 | |

qui est stérile par filtration à 0,2 micron, d'une osmolalité de 285 mOsM/kg et d'un pH de 7,17, où les phospholipides (S80) ne sont pas hydrogénés, et l'orthophosphate disodique dihydraté est utilisé comme système tampon.

**5.** Formulation liposomale ophtalmique selon la revendication 2 et 3 ayant la composition suivante :

|  | % p/p | Numéro CAS : |
|---|---|---|
| Curcumine | 0,050 | 458-37-7 |
| Phospholipides 90H | 1,000 | 97281-48-0 |
| VITAMINE E-TPGS | 0,050 | 9002-96-4 |
| Orthophosphate disodique dihydraté | 0,685 | 10028-24-7 |
| Orthophosphate monosodique dihydraté | 0,225 | 13472-35-0 |
| Chlorure de sodium | 0,570 | 7647-14-5 |
| Eau distillée si nécessaire pour | 100 | |

qui est stérile par filtration, d'une osmolalité de 286 mOsM/kg et d'un pH de 7,24, où les phospholipides (90H) sont hydrogénés, et l'orthophosphate disodique dihydraté est utilisé comme système tampon.

**6.** Formulation liposomale ophtalmique selon les revendications 2 et 3 ayant la composition suivante :

|  | % p/p | Numéro CAS : |
|---|---|---|
| Curcumine | 0,050 | 458-37-7 |
| Phospholipides 90H | 1,000 | 97281-48-0 |
| VITAMINE E-TPGS | 0,050 | 9002-96-4 |
| Acide borique | 0,775 | 10043-35-3 |
| TÉTRABORATE DE SODIUM DÉCAHYDRATÉ | 0,125 | 1303-96-4 |
| Chlorure de sodium | 0,450 | 7647-14-5 |
| Eau distillée si nécessaire pour | 100 | |

qui est stérile par filtration, d'une osmolalité de 286 mOsM/kg et d'un pH de 7,24, où les phospholipides (90H) sont hydrogénés, et l'acide borique est utilisé comme système tampon.

**7.** Formulation liposomale ophtalmique selon l'une quelconque des revendications précédentes pour une utilisation en gouttes ophtalmiques, après stérilisation par filtration.

**8.** Formulation liposomale ophtalmique selon l'une quelconque des revendications précédentes pour une utilisation après stérilisation par filtration, en gouttes ophtalmiques pour le traitement du syndrome de l'œil sec.

**9.** Procédé de préparation de la formulation liposomale ophtalmique contenant de la curcumine et de la Vit. E TPGS de l'une quelconque des revendications 1 à 6, **caractérisé en ce qu'**il comprend les étapes de fonctionnement suivantes :

i) la solubilisation de phospholipides contenant de la curcumine et la Vit. E TPGS dans un solvant organique pour l'inclusion spécifique du principe actif et de la Vit. E TPGS dans les phospholipides, et le séchage sous vide du mélange organique constitué des phospholipides, de la curcumine et de la Vit. E TPGS jusqu'à l'élimination complète des solvants ;

ii) la pâte sèche de phospholipides ainsi obtenue est hydratée dans de l'eau contenant le tampon et d'autres excipients pendant une durée d'hydratation appropriée, puis homogénéisée à l'aide d'un homogénéisateur à cisaillement pendant une période appropriée de temps pour obtenir un mélange de Pro-liposomes ;

iii) l'extrusion de la solution de Pro-liposomes à haute pression de $9,119*10^7$ Pa à $1,013*10^8$ Pa (de 900 à 1000 atm) pendant au moins 5 fois pour obtenir la taille souhaitée inférieure à 200 nm ;

iv) filtration en ligne à 0,2 micron de la solution de liposomes qui vient d'être extrudée ;

v) la collecte de la solution de liposomes stérilisée par filtration à 0,2 micron dans un récipient stérile.

10. Procédé de préparation de la formulation liposomale ophtalmique selon la revendication 9, **caractérisé en ce qu'**à l'étape i), des hydrogénés phospholipides sont utilisés.

11. Procédé de préparation de la formulation liposomale ophtalmique selon la revendication 9, **caractérisé en ce qu'**à l'étape i), des phospholipides non hydrogénés sont utilisés.

12. Composition liposomale ophtalmique de curcumine avec de la vitamine E-TPGS intercalée dès le départ dans un film lipidique pour obtenir une plus grande protection, obtenue selon le procédé de la revendication 9, pour une utilisation après filtration à 0,2 um, comme gouttes ophtalmiques pour le traitement topique de syndrome de l'œil sec.

FIG. 1

|  | | Size (d.nm): | % Intensity: | St Dev (d.nm): |
|---|---|---|---|---|
| Z-Average (d.nm): 82.27 | Peak 1: | 102.8 | 97.3 | 54.41 |
| PdI: 0.244 | Peak 2: | 4528 | 2.7 | 865.7 |
| Intercept: 0.950 | Peak 3: | 0.000 | 0.0 | 0.000 |
| Result quality : Good | | | | |

Size Distribution by Intensity

Intensity distribution average

FIG. 2

| | | | Size (d.nm): | % Intensity: | St Dev (d.nm): |
|---|---|---|---|---|---|
| Z-Average (d.nm): | 80.38 | Peak 1: | 101.7 | 98.8 | 57.45 |
| Pdi: | 0.228 | Peak 2: | 4740 | 1.2 | 756.1 |
| Intercept: | 0.950 | Peak 3: | 0.000 | 0.0 | 0.000 |
| **Result quality :** | Good | | | | |

Size Distribution by Intensity

Intensity distribution average

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102008439
- CN 102379850

- CN 1824255 A

### Non-patent literature cited in the description

- **DI PIERRO F et al.** Comparative evaluation of the pain-relieving properties of a lecithinized formulation of curcumin (Meriva®), nimesulide, and acetaminophen. *Journal of Pain Research,* 2013, 6201-205